**Europäisches Patentamt**

**European Patent Office**

**Office européen des brevets**

(19)

(11) Veröffentlichungsnummer: **0 280 982 B1**

(12) **EUROPÄISCHE PATENTSCHRIFT**

(45) Veröffentlichungstag der Patentschrift:
19.06.91 Patentblatt 91/25

(51) Int. Cl.⁵: **B01J 23/86, C07C 29/136,** C07C 31/125

(21) Anmeldenummer: **88102546.4**

(22) Anmeldetag: **22.02.88**

(54) Säureresistenter Katalysator für die Fettsäuredirekthydrierung zu Fettalkoholen.

(30) Priorität: **02.03.87 DE 3706658**

(43) Veröffentlichungstag der Anmeldung:
**07.09.88 Patentblatt 88/36**

(45) Bekanntmachung des Hinweises auf die
Patenterteilung:
**19.06.91 Patentblatt 91/25**

(84) Benannte Vertragsstaaten:
**AT BE CH DE ES FR GB IT LI LU NL SE**

(56) Entgegenhaltungen:
**EP-A- 0 023 699**
**DE-A- 1 005 497**
**DE-A- 2 613 226**
**FR-A- 889 791**
**FR-A- 1 276 722**
**US-A- 2 089 433**
**US-A- 3 899 577**

(73) Patentinhaber: **Henkel**
**Kommanditgesellschaft auf Aktien**
**Postfach 1100 Henkelstrasse 67**
**W-4000 Düsseldorf-Holthausen (DE)**

(72) Erfinder: **Pohl, Joachim, Dr.**
**Leinenweberweg 23**
**W-4000 Düsseldorf (DE)**
Erfinder: **Carduck, Franz-Josef, Dr.**
**Landstrasse 18**
**W-5657 Haan (DE)**
Erfinder: **Göbel, Gerd, Dr.**
**Lindenstrasse 10**
**W-4006 Erkrath (DE)**

**Beschreibung**

Die vorliegende Erfindung betrifft die Bereitstellung eines neuen säureresistenten Kupferchromit-Spinellkatalysators für die Fettsäuredirekthydrierung zu Fettalkoholen.

Fettalkohole, d.h. überwiegend lineare, monofunktionelle Alkohole mit Kettenlägen von 8 und mehr C-Atomen, sowie ihre Herstellung sind in der Literatur ausführlich beschrieben, beispielsweise in Ullmanns Encyklopädie der technischen Chemie, 4. Auflage, Band 11, Seiten 427 bis 445. Ein bevorzugtes Ausgangsmaterial zu ihrer Gewinnung sind die in Fetten und/oder Ölen natürlichen Ursprungs vorkommenden Fettsäuren bzw. Fettsäuregemische, die durch katalytische Hydrierung in Fettalkohole entsprechender Kettenlänge umgewandelt werden können. Durch den Einsatz der zu reduzierenden Fettsäuren in Form ihrer Methylester werden insbesondere die Katalysatoren vor einem aggressiven Angriff durch die freie Carboxylgruppe geschützt, so daß in großtechnischen Verfahren für hinreichend lange Zeiträume mit befriedigenden Raumzeitausbeuten gearbeitet werden kann. Demgemäß wird die überwiegende Menge nativer Fettalkohole heute aus Fettsäuremethylestern hergestellt, bei dem die destillierten Methylester über fest angeordnete, kupferhaltige Mischoxid-Katalysatoren, wie beispielsweise Kupfer-/Zink-Katalysatoren, in flüssigem Zustand zusammen mit einem großen Überschuß an Wasserstoff bei Temperaturen von oberhalb 200°C und Drucken von etwa 250 bis 300 bar geleitet werden.

Die auf feuchtem Wege durch gemeinsame Fällung hergestellten Kupfer-Mischoxid-Katalysatoren werden als stückige Kontakte oder Stangenpreßlinge benutzt und vor Gebrauch meist in der Anlage reduziert. Sie sind nicht säurefest. Die Gewinnung nativer Fettalkohole durch direkte Hydrierung der freien Fettsäuren kann dementsprechend bis heute kaum praktisch angewendet werden.

Nach Ullmann, loc. cit., ist es bekannt, daß die Hydrierung freier Fettsäuren zu Fettalkoholen im Suspensionsverfahren mit Kupfer-II-chromit-Katalysatoren vorgenommen werden kann. Diese Arbeitsweise läßt sich allerdings nur dann einigermaßen brauchbar einsetzen, wenn der Kupfer-II-chromit-Katalysator durch Zersetzung des primär gewonnenen Kupferammoniumchromat-Komplexes und anschließende Wäsche mit Eisessig gewonnen wird. Derartig hergestellte Katalysatoren sind besonders teuer und praktisch nur für die Suspensionshydrierung verwendbar. Säuregewaschenes Kupfer-II-chromit ist nicht oder nur sehr schlecht tablettierbar und dementsprechend nicht in abriebfeste oder mechanisch feste Strangpreßlinge oder andere Formkörper zu überführen. Versucht man, diese Verfestigung durch Temperung zu erreichen, vermindert sich die katalytische Wirkung. Auch der Versuch, säuregewaschenes Kupfer-II-chromit auf Katalysatorträger, wie beispielsweise Silicagel oder Aluminiumoxid, aufzubringen, führt nicht zu technisch verwertbaren Katalysatoren. Der Träger wird angegriffen und der Katalysator wird vom Träger leicht heruntergewaschen.

In der einschlägigen Patentliteratur werden daher häufig Fettsäureester, insbesondere Fettsäuremethylester und freie Fettsäuren gleichzeitig als Einsatzmaterialien für die Hydrierungsreaktion zu gesättigten und /oder ungesättigten Fettalkoholen verwendet. Verwiesen sei beispielsweise auf die DE-C 965 236, DE-B 10 05 497, DE-A 25 13 377 und 26 13 226. Für die großtechnische praktische Verwertung sind die genannten Vorschläge durch-aus unterschiedlich zu bewerten, je nachdem, ob die Fettsäureester oder die freien Fettsäuren als Ausgangsmaterial der Hydrierung verwendet werden. Allgemein bekannt ist, daß die großen Vorteile der Festbettkatalyse mit Massivkontakten für die Verarbeitung eines aus freien Fettsäuren bestehenden oder freie Fettsäuren enthaltenden Ausgangsmaterials nicht erschlossen werden konnten. Der korrosive Angriff der freien Säuren bei hohen Temperaturen und Drucken auf die in der Methylesterreduktion an sich bewährten Massivkontakte ist so stark, daß eine praktisch-technische Verwertung der genannten Vorschläge für die Reduktion freier Säuren bisher nicht in Betracht kam.

In der Praxis behilft man sich daher, indem man im Hydrierreaktor fertigen Fettalkohol in größerer Menge vorlegt und später unter Hydrierbedingungen freie Fettsäure zugibt. Das Verfahren erfordert jedoch unverhältnismäßig große Reaktoren. Es erreicht nur Umsetzungsgrade von 96%, während festbettkatalytische Verfahren 99%ige Umsetzgrade und mehr erreichen.

Kupferchromit-Katalysatoren sind, wie oben ausgeführt, hochaktive Kontakte für die Hydrierung von Fettsäureestern und Triglyceriden von Ölen und Fetten. Die direkte Hydrierung von Fettsäuren mit dieser Katalysatorklasse war bislang nicht möglich, da Kupfer-II-chromit ($CuCr_2O_4$) nicht säurestabil ist, d.h. durch die Einwirkung der freien Fettsäure gelöst wird. Dies gilt insbesondere für das im Katalysator enthaltene CuO.

Aufgabe der vorliegenden Erfindung ist es daher, Kupfer-II-chromit bei hohen Temperaturen in eine säurefeste Spinellform zu überführen, unter Beibehaltung einer möglichst hohen spezifischen Katalysatoroberfläche und der entsprechenden Porenstruktur, die eine hohe Aktivität und Raumzeitausbeute gewährleistet.

Gegenstand der vorliegenden Erfindung ist somit ein Verfahren zur Herstellung eines säureresistenten Kupferchromit-Spinellkatalysators zur Festbetthydrierung von Fettsäuren zu Fettalkoholen entsprechender Kettenlänge, wobei man Kupfer-II-chromit ($CuCr_2O_4$) unter Verwendung von kolloidalem Kieselgel in an sich bekannter Weise darstellt, das dadurch gekennzeichnet ist, daß man das hergestellte Kupfer-II-chromit bei

2

einer Temperatur von wenigstens 750°C im Verlauf von wenigstens 12 h glüht.

Einer Ausführungsform der vorliegenden Erfindung entsprechend kann man das calcinierte pulverförmige Katalysatormaterial mit 0,5 bis 10 Gew.-% eines organischen Bindemittels, wie beispielsweise Polyvinylacetat oder Methylmethacrylat und 0,5 bis 15 Gew.-% Graphit vermischen, granulieren und anschließend zu Tabletten verpressen.

Weiterer Gegenstand der Erfindung ist der säureresistente Kupfer-II-chromit-Spinellkatalysator, der dadurch gekennzeichnet ist, daß er einen Kieselgelgehalt von 0,1 bis 15 Gew.-% aufweist.

Eine besondere Ausführungsform des erfindungsgemäßen Kupferchromit-Spinellkatalysators besteht darin, daß der Kieselgelgehalt 4,5 Gew.-%, bezogen auf den fertigen Gesamtkontakt, beträgt.

Der erfindungsgemäße Katalysator ist geeignet zur direkten Festbetthydrierung von Fettsäuren zu Fettalkoholen entsprechender Kettenlänge.

Gemäß einer Ausführungsform der vorliegenden Erfindung kommen als Einsatzgut Fettsäuregemische mit 6 bis 24 C-Atomen in Frage, die insbesondere aus tierischen und/oder pflanzlichen Fetten und/oder Ölen gewonnen werden können. Das Direkthydrierverfahren gemäß der vorliegenden Erfindung ist dadurch gekennzeichnet, daß man mit einem Volumenverhältnis von Katalysatormenge zu Fettsäuregemisch im Bereich von 0,1 bis 3 pro Stunde arbeiten kann.

Gemäß der vorliegenden Erfindung ist es somit möglich, die an sich bekannte Säurestabilität des Kupfer-II-Spinells für die großtechnische Direkthydrierung der Fettsäuren zu Fettalkoholen auszunutzen, ohne gleichzeitig Raumzeitausbeute-Verluste hinzunehmen. Die schlechten Raumzeitausbeuten, die für Kupfer-I-Spinelle in der Literatur angegeben werden, konnten durch die Aufrechterhaltung der Porosität des Spinells gemäß dem vorliegenden Verfahren in unerwarteter Weise verbessert werden.

Bei den Untersuchungen der neuen Katalysatoren wurde gefunden, daß bei einem $SiO_2$-Gehalt im Katalysator hinsichtlich der Aktivität und der Säureresistenz ein Optimum durchlaufen wird, wenn der Gehalt an $SiO_2$ 4,5 Gew.-%, bezogen auf den fertigen Gesamtkontakt, beträgt.

Im Gegensatz zu dem Kupfer-II-chromit-Katalysator ist der erfindungsgemäße Kupfer-II-Spinellkatalysator gut tablettierbar und läßt sich somit als Festbettkatalysator einsetzen.

Das erfindungsgemäße Verfahren zur direkten katalytischen Hydrierung von Fettsäuren konnte überraschenderweise auch dadurch verbessert werden, daß durch eine Optimierung nicht nur der chemischen Zusammensetzung des Katalysators, die oben beschrieben wurde, sondern auch des physikalischen Aufbaus der Formkörper des Katalysators eine Erhöhung der Katalysatorwirksamkeit im Vergleich zu handelsüblichen Katalysatoren erreicht wurde. Darunter ist zu verstehen, daß der physikalische Aufbau der Katalysator-Formkörper überraschenderweise ebenfalls erhebliche Auswirkungen auf die Aktivität und Selektivität des Katalysators hat. Erfindungsgemäß wurde eine Verbesserung des Verfahrens dadurch erreicht, daß der verwendete Katalysator unter Einsatz von 1 bis 10 Gew.-% eines oder mehrerer Binder in Tablettenform gebracht wird. Bevorzugt wird dabei ein Einsatz von 1 bis 5 Gew.-% eines oder mehrerer Binder. Als solche kommen für diesen Zweck aus dem Stand der Technik bekannte Verbindungen in Frage, wobei in dem erfindungsgemäß verwendeten Katalysator entweder ein oder auch mehrere Binder verwendet werden können. Besonders hat sich der Einsatz eines oder mehrerer Binder aus der Gruppe Polyvinylacetat und Methylmethacrylat bewährt. Bevorzugt wird als Binder für die Herstellung der Katalysatortabletten Polyvinylacetat, wobei zur Katalysatorherstellung beispielsweise 10 Gew.-%ige Polyvinylacetat-Suspensionen verwendet werden, die im Handel kommerziell erhältlich sind. Die calcinierten pulverförmigen Katalysatormaterialien werden mit derartigen Polyvinylacetat-Suspensionen in kleinen Mengen versetzt und bis zum Beginn des Aufbaus von Agglomeratkörnern gemischt. Im Anschluß daran wird das Agglomerate enthaltende Pulver beispielsweise in einem Lochwalzengranulator zu kleinen Granulaten verdichtet. Auf an sich bekannte Weise werden die Granulate getrocknet, wobei sich Restfeuchten von 10 bis 15% einstellen lassen. Die aus diesem Vorgang resultierenden Granulate werden gesiebt und zu Tabletten verpreßt.

Die in dem erfindungsgemäßen Verfahren verwendeten Katalysatoren werden üblicherweise vor ihrem Einsatz in der Direkthydrierung von Fettsäuren mit Wasserstoff oder einem Wasserstoff enthaltenden Gasgemisch aktiviert. Mit Vorteil wird zur Aktivierung der Katalysatormassen ein Gasgemisch verwendet, das überwiegend aus einer Stickstoff-/Wasserstoff-Gasmischung besteht. Vorteilhafterweise kann eine derartige Aktivierung – wie aus dem Stand der Technik bekannt – so durchgeführt werden, daß die Katalysatormassen nach der Herstellung im Stickstoffstrom bei erhöhter Temperatur getrocknet werden und dem trocknenden Gas in steigenden Mengen Wasserstoff zur Aktivierung beigemischt wird. Dabei kann der Wasserstoffanteil in dem aktivierenden Gasgemisch im Bereich von 0,1 bis 10 Vol.-% liegen. Die Aktivierung der Katalysatoren kann dabei sowohl in situ als auch in von dem Reaktionsgefäß getrennten Gefäßen durchgeführt werden.

Es ist bekannt, daß native Fett und Öle, je nach Herkunft, kleinere oder auch größere Anteile an einfach oder auch mehrfach olefinisch ungesättigten Fettsäuren enthalten. Da das Kupfer auch Doppelbindungen absättigt, entstehen auch aus ungesättigten Fettsäureestern gesättigte Alkohole.

Über die Kontrolle der Beschaffenheit des Verfahrensproduktes läßt sich leicht – in Abstimmung mit dem Verhältnis von Kreislaufwasserstoff zu Einsatzgut – die richtige Temperatur wählen. Zu niedrige Verfahrenstemperaturen führen zu korrosivem Angriff auf den Festbettkatalysator und damit zum Austrag von Metallseifen über das Reaktionsprodukt. Es können dabei sowohl Chromseifen als auch Kupferseifen anfallen. Zu hohe Verfahrenstemperaturen führen zu einer Überreduktion und damit zur unerwünscht hohen Bildung von Paraffinen.

Die im Einzelfall einzustellenden Reaktionsparameter werden u.a. durch die Länge der Kohlenstoffketten der zu reduzierenden Fettsäuren oder Fettsäuregemische mitbestimmt. Je kürzer die Kettenlänge der eingesetzten Fettsäuren ist, umso niedriger sind im allgemeinen die Reaktionstemperaturen innerhalb der angegebenen Bereiche.

Prinzipiell können erfindungsgemäß verwendeten Fettsäure-Einsatzmaterialien zur Modifizierung der Verfahrensbedingungen niedere Alkohole, leichtsiedende Paraffine oder Wasserdampf, zugesetzt werden. Es muß jedoch dafür gesorgt werden, daß auch die entstandenen gasförmigen Reaktionsnebenprodukte aus dem Reaktionskreislauf und insbesondere aus dem Kreislaufgas ausgeschleust werden. In Betracht kommen hier insbesondere das bei der Reaktion entstehende Wasser, geringe Mengen an Kohlenwasserstoffen sowie die über Wasserstoffnachspeisung unvermeidlich eingeführten Mengen an Stickstoff.

Die Verfahrensdrucke liegen im allgemeinen bei 200 bar und darüber, insbesondere im Bereich von 200 bis 500 bar. Prinzipiell führt die Verwendung von höheren Drucken zur Senkung der Säurezahl der Reaktionsprodukte und damit zu einer Steigerung der Ausbeute an erwünschten Fettalkoholen.

Im folgenden Beispiel 1 wird die Herstellung eines besonders geeigneten Kupfer-II-Spinells angegeben, der in den nachfolgenden Beispielen 2 und 3 eingesetzt wird.

## Beispiel 1

85 g Bariumnitrat, 294 g Mangannitrat und 2493 g Kupfernitrat werden in 9 l entsalztem Wasser gelöst. Zur klaren Lösung werden 550 g eines 40 %igen $SiO_2$-Kolloids zugegeben. Die Mischung wird auf 70°C erhitzt.

In einem zweiten Rührbehälter werden 1639 g Chromsäure in 9 l Wasser gelöst. Zu dieser Lösung werden 3600 g einer 25 %igen Ammoniaklösung hinzugegeben. Die Lösung wird auf 70°C aufgeheizt. Die Fällung des Kupferchromats erfolgt durch die Zugabe der Cu, Ba, Mn-Nitrat-lösung zu der vorgelegten Ammonchromatlösung. Der Filterkuchen wird nitratfrei gewaschen und getrocknet. Die Kalzinierung erfolgt 12 Stunden bei 750°C. Danach wird das Pulver mit 2% Polyvinylacetat und 2% Graphit vermischt, granuliert und zu 4 × 4 mm Tabletten verpreßt.

## Beispiel 2

12 g Pulver des gemäß Beispiel 1 hergestellten Katalysators und 600 g $C_{12}$-Fettsäure werden bei einer Temperatur von 260°C und einem Wasserstoffdruck von 250 bar in einem 2 l Autoklaven zur Reaktion gebracht. Die Umsätze der Fettsäure wurden nach 1, 3 und 5 Stunden wie folgt ermittelt :

| Reaktionsdauer (h): | 1 | 3 | 5 |
|---|---|---|---|
| FS-Umsatz (%) : | 39 | 48 | 75 |

Der Katalysator wurde bei der Hydrierung nicht gelöst.

## Beispiel 3

Tabletten (4 × 4 mm) im Volumen von 500 ml werden in einem Rohrrekator mit einem $H_2/N_2$-Gemisch ($H_2$: $N_2$ = 1 : 10) bei 200°C reduziert. Der Katalysator wird vorsichtig mit $C_{12/18}$ Fettsäuremethylester angefahren. Anschließend werden bei einem Wasserstoffdruck von 250 bar und einer Temperatur von 260 bis 275°C 400 bis 500 l $C_{12}$-Fettsäure pro Stunde über der Katalysator gepumpt. Die Abläufe sind wasserklar. Es wurden folgende Kennzahlen ermittelt :

Säurezahl:        0,04
Verseifungszahl:  um 2
Hydroxylzahl:     285 bis 293


**Ansprüche**

1. Verfahren zur Herstellung eines säureresistenten Kupferchromit-Spinellkatalysators zur direkten Festbetthydrierung von Fettsäuren zu Fettalkoholen entsprechender Kettenlänge, wobei man Kupfer-II-chromit (CuCr$_2$O$_4$) unter Verwendung von kolloidalem Kieselgel in an sich bekannter Weise darstellt, dadurch gekennzeichnet, daß man das hergostellte Kupfer-II-chromit bei einer Temperatur von wenigstens 750°C im Verlauf von wenigstens 12 h glüht.

2. Verfahren zur Herstellung eines säureresistenten Kupfer-II-chromit-Spinellkatalysators nach Anspruch 1, dadurch gekennzeichnet, daß man das calcinierte pulverförmige Katalysatormaterial mit 0,5 bis 10 Gew.-% eines organischen Bindemittels, vorzugsweise Polyvinylacetat, und 0,5 bis 15 Gew.-% Graphit vermischt, granuliert und zu Tabletten verpreßt.

3. Säureresistenter Kupfer-II-chromit-Spinellkatalysator nach Ansprüchen 1 und 2, dadurch gekennzeichnet, daß er einen Kieselgehalt von 0,5 bis 15 Gew.-%, bezogen auf den fertigen Gesamtkontakt, aufweist.

4. Säureresistenter Kupfer-II-chromit-Spinellkatalysator nach Anspruch 3, dadurch gekennzeichnet, daß er einen Kieselgelgehalt von 4,5 Gew.-%, bezogen auf den fertigen Gesamtkontakt, aufweist.

5. Verwendung eines säureresistenten Kupfer-II-chromit-Spinellkatalysators nach den Ansprüchen 1 bis 4 zur direkten Festbetthydrierung von Fettsäuren zu Fettalkoholen entsprechender Kettenlänge.

6. Verwendung nach Anspruch 5, dadurch gekennzeichnet, daß man als Einsatzgut Fettsäuregemische mit 6 bis 24 C-Atomen einsetzt, die insbesondere aus tierischen und/oder pflanzlichen Fetten und/oder Ölen gewonnen worden sind.

7. Verwendung nach Anspruch 6, dadurch gekennzeichnet, daß man mit einem Volumenverhältnis von Katalysatormenge zu Fettsäuregemisch im Bereich von 0,1 bis 3 pro Stunde arbeitet.


**Claims**

1. A process for the production of an acid-resistant copper chromite spinel catalyst for the fixed-bed hydrogenation of fatty acids to fatty alcohols of corresponding chain length, copper (II) chromite (CuCr$_2$O$_4$) being prepared in known manner using colloidal silica gel, characterized in that the copper (II) chromite produced is calcined for at least 12 h at a temperature of at least 750°C.

2. A process for the production of an acid-resistant copper (II) chromite spinel catalyst as claimed in claim 1, characterized in that the calcined powder-form catalyst material is mixed with from 0.5 to 10% by weight of an organic binder, preferably polyvinyl acetate, and 0.5 to 15% by weight graphite, the resulting mixture is granulated and then tabletted.

3. An acid-resistant copper (II) chromite spinel catalyst according to claims 1 and 2, characterized in that it has a silica gel content of from 0.5 to 15% by weight, based on the final catalyst as a whole.

4. An acid resistant copper (II) chromite spinel catalyst according to claim 3, characterized in that it has a silica gel content of 4.5% by weight, based on the final catalyst as a whole.

5. The use of an acid-resistant copper (II) chromite spinel catalyst according to claims 1 to 4 for the direct fixed-bed hydrogenation of fatty acids to fatty alcohols of corresponding chain length.

6. The use claimed in claim 5, characterized in that C$_{6-24}$ fatty acid mixtures obtained in particular from animal and/or vegetable fats and/or oils are used as starting material.

7. The use claimed in claim 6, characterized in that it is carried out using a ratio by volume of the quantity of catalyst to fatty acid mixture of from 0.1 to 3 per hour.


**Revendications**

1. Procédé de fabrication d'un catalyseur à base de spinelle cupro-chromique stable en milieu acide, destiné à l'hydrogénation directe en milieu solide d'acides gras pour obtenir des alcools gras de longueur de chaîne correspondante, dans lequel, on prépare du chromate de cuivre II ($CuCr_2O_4$) en utilisant du gel de silice d'une façon connue en, caractérisé en ce que l'on porte à l'incandescence à une température d'au moins 750°C pendant au moins 12 heures le chromate de cuivre II obtenu.

2. Procédé de fabrication d'un catalyseur à base de spinelle cupro II chromique selon la revendication 1, caractérisé en ce que l'on mélange la substance du catalyseur sous forme de poudre calcinée avec 0,5 à 10% en poids d'un agent liant organique, préférentiellement de l'acétate de polyvinyle, et 0,5 à 15% en poids de graphite, on réduit à l'état de granulés, et l'on compacte sous forme de pastilles.

3. Catalyseur à base de spinelle cupro II chromique résistante aux acides selon les revendications 1 et 2, caractérisé en ce qu'il présente une teneur en silice de 0,5 à 15% en poids, mesurée par rapport à l'ensemble de la masse des sites de contact préparée.

4. Catalyseur à base de spinelle cupro II chromique résistante aux acides, caractérisé en ce qu'il présente une teneur en silice de 4,5% en poids, mesurée par rapport à l'ensemble de la masse des sites de contact préparée.

5. Utilisation d'un catalyseur à base de spinelle cupro II chromique résistante aux acides selon les revendications 1 à 4 pour l'hydrogénation directe en milieu solide d'acides gras pour obtenir des alcools gras en longueur de chaîne correspondante.

6. Utilisation selon la revendication 5, caractérisée en ce qu'on utilise comme matières premières des mélanges d'acides gras comportant 6 à 24 atomes de carbone, provenant notamment de graisses animales et/ou végétales et/ou d'huiles.

7. Utilisation selon la revendication 6, caractérisée en ce que l'on effectue la réaction avec un rapport volumique entre la quantité de catalyseur et le mélange d'acides gras (traités) compris entre 0,1 et 3 par heure.